# EUROPEAN PATENT SPECIFICATION

(11) **EP 0 442 423 B1**
(45) Date of publication and mention of the grant of the patent: **11.01.1995**
(21) Application number: 91101888.5
(22) Date of filing: 11.02.1991
(51) Int. Cl.: A61K 31/505

(54) **Use of buspirone in sleep apneas**
Verwendung von Buspiron in Schlaf-Apnoe
Utilisation de la buspirone dans les apnées de sommeil

(30) Priority: 12.02.1990 US 479803
(43) Date of publication of application: 21.08.1991
(73) Proprietor: Bristol-Myers Squibb Company, New York, N.Y. 10154 (US); STANFORD, UNIVERSITY, Stanford, California 94305 (US)
(72) Inventor: Dement, William C., Stanford, California 94305 (US); Rosekind, Mark R., Los Altos, California 94022 (US); Schwimmer, Jeffrey L., Morristown, New Jersey 07960 (US)
(74) Representative: Kinzebach, Werner, Dr.

(56) References cited:
- FASEB J., vol. 2, no. 5, 1988, page A1507, abstract no. 7030; D.M. RAPOPORT etal.: "Comparison of the effects of buspirone and diazepam on control of breathing"
- AM. REV. RESPIR. DIS., vol. 139, no. 4, April 1989, pages 946-950; S.J.GARNER et al.: "Buspirone, an anxiolytic drug that stimulates respiration"
- AM. REV. RESPIR. DIS., vol. 139, no. 4, April 1989, page A625; D.M. RAPOPORT etal.: "Buspirone - A new respiratory stimulant"
- AM. REV. RESPIR. DIS., vol. 141, no. 6, June 1990, pages 1527-1530; W.B.MENDELSON et al.: "Effects of buspirone on sleep and respiration"
- DRUG. THER., vol. 20 suppl., August 1990, pages 52-57; M.B. SCHARF: "Sleep inaffective disorders"
- BRAIN RES., vol. 535, no. 2, 10th December 1990, pages 281-287; D. MORIN et al.: "Serotonergic influences on central respiratory activity: an in vitro studyin the newborn rat"
- "The Merck Manual of Diagnosis and Therapy", 15th edition, 1987, pages 1379-1380, Merck & Co., Inc., Rahway, N.J., US; T. BERKOW et al.:"Sleep Apnea"

## Description

This invention is concerned with the use of the psychotropic agent, buspirone. Buspirone is a member of the azapirone class of psychotropic agents and has the following structural formula 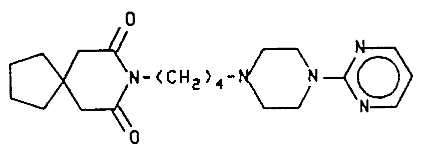
The hydrochloride salt has been referred to in the prior art as MJ 9022-1 and as buspirone hydrochloride. Other acid addition salts thereof are named by combining "buspirone" with the appropriate word to define the acid from which it is prepared as in "buspirone hydrochloride". The latter is the United States Adopted Name (USAN); refer to J. American Med. Assoc. 225, 520 (1973).

The synthesis of the compound and the disclosure of its psychotropic properties are described in the following patents and publications.
1. Y.H. Wu, et al., J. Med. Chem., 15,477 (1972).
2. Y.H. Wu, et al., U.S. Pat. No. 3,717,634 which issued February 20, 1973.
3. L.E. Allen et al., Arzneium. Eorsch., 24, No. 6, 917-922 (1974).
4. G.L. Sathananthan, et al., Current Therapeutic Research, 18/5, 701-705 (1975).
5. Y.H. Wu, et al., U.S. Pat. No. 3,976,776, issued August 24, 1976.

The following patent references disclose and claim additional uses that relate to buspirone's pharmacological effects on the central nervous system.
6. The use of buspirone hydrochloride as a novel antianxiety agent for the treatment of neurotic patients is described in G.P. Casten, et al., U.S. Patent No. 4,182,763, issued January 9, 1980.
7. Allen, et al., disclose the use of buspirone in treating extrapyramidal motor disorders in U.S. 4,438,119, issued March 20, 1984.
8. Buspirone's use in sexual dysfunction was described by Othmer, et al., in U.S. 4,640,921, issued February 3, 1987.
9. Kurtz, et al., in U.S. 4,634,703, issued January 6, 1987 disclose buspirone's use in treating panic disorders.
10. Alderdice discloses the use of buspirone in the improvement of short term memory in U.S. 4,687,772, issued August 18, 1987.
11. U.S. 4,777,173, issued October 10, 1988 to Shrotriya and Casten, discloses and claims the use of buspirone in treating alcohol abuse.

The present invention resulted from the discovery that buspirone was effective in treating patients suffering from sleep apneas. The only literature relating to the effects of buspirone respiration were general disclosures that buspirone had a stimulatory effect on respiration both in cats (Garner, et al., Am. Rev. Respir. Dis., 139:4, pages 946-950 (1989) and D.M. Rapoport et al. ibid., 139, A 625 (1989)) and in normal male volunteers (Rapoport, et al., Fed. Am. Soc. Exp. Biol., J 2:5, Abstract 7030 (1988).

It has become apparent that sleep apneas comprise a spectrum of related disorders with varying severity and morbidity. Sleep apneas have been usually classified as being an obstructive, central, or mixed apnea, depending on the presence or absence of respiratory efforts during the periods in which airflow has ceased. Obstructive and mixed apneas occur with the greatest frequency. The most familiar of these is obstructive sleep apnea syndrome in which sporadic recurring collapse of the patient's upper airway occurs during sleep. If the collapse is complete there is no air exchange at the nose and mouth and breathing is interrupted. The usual result is a partial arousal from sleep and a return to normal breathing. The patient in most instances has no knowledge or memory of these apnea episodes but finds himself constantly suffering from fatigue and daytime sleepiness for no apparent reason. These recurrent apnea episodes with resultant hypoxemia and fragmented sleep can have serious neurologic and cardiac consequences. In recent years there has been a growing awareness of the seriousness of the sleep apnea problem due to its wide occurrence and the lack of a recognized effective treatment.

Surgical and mechanical interventions as well as oxygen administration have been employed as treatments. None of these are very suitable. Treatment of sleep apneas by pharmacological intervention has also had little success. Respiratory stimulants, theophylline, antidepressants and progestational agents have been used to treat sleep apneas but have not been found to be very effective.

In sum, there is nothing in the prior art, including the specific references set forth hereinabove, that would suggest the use of buspirone for the treatment of sleep apneas.

The present invention relates to the use of buspirone, or a pharmacologically acceptable acid addition salt therof for preparing a pharmaceutical composition for treatment of sleep apneas. For this purpose, buspirone or a pharmacologically acceptable acid addition salt therof is administered to a patient in need of such treatment. Oral administration of a dose of from about 10 to 60 mg of buspirone at the hour of sleep is usually employed.

The invention results from the discovery that buspirone administration is an effective treatment in preventing or reducing the incidence of sleep apneas. In the context of this invention, sleep apneas comprise all the sub-categories such as those caused by upper airway obstruction; those whose origins arise in the central nervous system; and those of a mixed type with contribution from both components. This invention is also intended for use in "sudden infant death syndrome" (SIDS). It should be appreciated that no established therapy for sleep apneas exists. As an example, treatment modalities for the most common subtype, chronic obstructive sleep apnea, have included weight loss, surgical removal of tissue that may cause obstruction, avoidance of all sedatives, mechanical interventions and oxygen masks. None of these measures has been effective in any consistent manner. In this as well as other sleep apneas, pharmacologic intervention has been used but effectiveness has not been established. Respiratory stimulants, antidepressants, theophylline and various progestational agents have been tried but not found to be very effective.

A recent review (Guilleminault, "Obstructive Sleep Apnea Syndrome", in Psychiatric Clinics of North America -Vol. 10. No. 4. pages 607-618 (1987)) reveals a widespread occurrence as well as a range of serious medical sequalae.

There are two aspects to the use of buspirone in treating sleep apneas. The first is that the administration of buspirone effectively reduces the frequency and severity of the apnea episodes during sleep. This is reflected in significantly increased undisturbed sleep and a significant increase in blood oxygen levels. The second aspect involves alleviation of the symptomatology associated with the occurrence of sleep apneas. The buspirone treatment alleviates the sleep apnea-related symptoms of anxiety, depression, fatigue, malaise, irritability, anger and hostility.

Clinical experience with buspirone would indicate that maximum levels of response to buspirone administration may be achieved with chronic administration, in some instances on the order of two to four weeks. It is appreciated by those skilled in the art that the time to emergence of full therapeutic response, as well as the dosage level required, can vary from patient to patient.

The effectiveness of buspirone treatment of patients suffering from sleep apneas has been exemplified by clinical experience. Single dose administration of buspirone, given at bedtime to patients suffering from obstructive sleep apnea, resulted in increased sleep efficiency with experimentally derived measurements showing a gain in total sleep time and a marked reduction in episodes of sleep disturbance. One of the most consistent physiological measurements of improvement was a 10 to 20% increase in blood oxygen levels, an indication of improved respiratory efficiency.

In summary, the present invention concerns the treatment of sleep apneas comprising obstructive, central and mixed apneas, in a patient population that ranges from infants to geriatric-aged individuals. This involves administration of buspirone, or one of its pharmaceutically acceptable salts, in the form of a pharmaceutical composition, either alone or as an adjunct to their therapies.

Pharmaceutically acceptable acid addition salts of buspirone and methods of pharmaceutical formulation are described in the patents of Wu, et al., U.S. 3,717,634 and U.S. 3,976,776.

Administration of buspirone according to the present invention may be made by the parenteral, oral, or rectal routes. Parenteral administration comprises injection, e.g., intravenous or intramuscular injection, as well as any other parenteral route of administration. The oral route is preferred. The clinical dosage range for treatment of sleep apneas depend on age of recipient, body weight, general physical condition and severity of sleep apnea disorder. In general the drug treatment will be administered at the hour of sleep and will be about 20 to 60 mg for an average adult. Dosage will be appropriately reduced for infants and young children, according to the clinical judgement of the attending physician.

## Claims

1. Use of buspirone or a pharmaceutically effective acid addition salt thereof for preparing a pharmaceutical composition for treatment of sleep apneas.

2. The use of claim 1 wherein buspirone hydrochloride is employed and dosage is by the oral route.

## Patentansprüche

1. Verwendung von Buspiron oder eines pharmazeutisch wirksamen Säureadditionssalzes davon zur Herstellung eines pharmazeutischen Mittels zur Behandlung von Schlafapnoe.

2. Verwendung nach Anspruch 1, wobei man Buspironhydrochlorid einsetzt und die Verabreichung auf oralem Weg erfolgt.

## Revendications

1. Utilisation de buspirone ou de son sel d'addition d'acide pharmaceutiquement efficace pour la préparation d'une composition pharmaceutique pour le traitement des apnées de sommeil.

2. Utilisation de la revendication 1, où on emploie le chlorhydrate de buspirone et le dosage est par voie orale.
